# EUROPÄISCHE PATENTSCHRIFT

(11) **EP 0 918 865 B1**
(45) Veröffentlichungstag und Bekanntmachung des Hinweises auf die Patenterteilung: **16.03.2005**
(21) Anmeldenummer: 97935563.3
(22) Anmeldetag: 01.08.1997
(51) Int. Cl.: C12N 15/31, C07K 14/20, G01N 33/50, A61K 39/02, C12Q 1/68, A61K 31/70

(54) **IMMUNOLOGISCH AKTIVE PROTEINE VON BORRELIA BURGDORFERI, DAFÜR KODIERENDE NUKLEINSÄUREN SOWIE DEREN VERWENDUNG IN TESTKITS UND ALS IMPFSTOFFE**
IMMUNOLOGICALLY ACTIVE PROTEINS OF BORRELIA BURGDORFERI, CODED NUCLEIN ACIDS OF SUCH AND THEIR USE IN TEST KITS AND VACCINES
PROTEINES DE BORRELIA BURGDORFERI IMMUNOLOGIQUEMENT ACTIVES, ACIDES NUCLEIQUES CODANT POUR CES PROTEINES, ET LEUR APPLICATION DANS LES NECESSAIRES D'ESSAI ET LES VACCINS

(30) Priorität: 14.08.1996 DE 19632862
(43) Veröffentlichungstag der Anmeldung: 02.06.1999
(73) Patentinhaber: Mikrogen Molekularbiologische Entwicklungs-GmbH, 82152 Martinsried (DE)
(72) Erfinder: MOTZ, Manfred, D-80689 München (DE); SOUTSCHEK, Erwin, D-82335 Berg (DE)
(74) Vertreter: Keller, Günter, Dr.
(86) Internationale Anmeldenummer: PCT/EP1997/004215
(87) Internationale Veröffentlichungsnummer: WO 1998/006850

(56) Entgegenhaltungen:
- WO-A-96/34106
- WO-A-97/27301
- GUO B P ET AL: "EVIDENCE THAT THE DECORIN BINDING PROTEIN OF BORRELIA BURGDORFERI IS AN ADHESIN" ABSTRACTS OF THE GENERAL MEETING OF THE AMERICAN SOCIETY FOR MICROBIOLOGY, Bd. 96, Mai 1996, Seite 248 XP000618881
- GUO B ET AL: "Identification of decorin binding proteins on the outer membrane surface of Borrelia burgdorferi" ABSTRACTS OF THE GENERAL MEETING OF THE AMERICAN SOCIETY FOR MICROBIOLOGY, 94 (0). MAI 1994. 124., XP002012437

### SEQUENZPROTOKOLL

(1) ALLGEMEINE INFORMATION:
   ANMELDER:
      (A) NAME: Mikrogen GmbH
      (B) STRASSE: Westendstr. 125
      (C) ORT: München
      (E) LAND: Germany
      (F) POSTLEITZAHL: 80339
   ANMELDETITEL:
      Immunologisch aktive Proteine von Borrelia burgdorferi, dafür kodierende Nukleinsäuren sowie deren Verwendung in Testkits und als Impfstoffe
   ANZAHL DER SEQUENZEN: 13
   COMPUTER-LESBARE FORM:
      (A) DATENTRÄGER: Floppy Disk
      (B) COMPUTER: IBM PC compatible
      (C) BETRIEBSSYSTEM: PC-DOS/MS-DOS
      (D) SOFTWARE: PADAT Sequenzmodul Version 1.0
(2) INFORMATION ZU SEQ ID NO: 1:
   (i) SEQUENZ CHARAKTERISTIKA:
      (A) LÄNGE: 183 Aminosäuren
      (B) ART: Aminosäure
      (C) STRANGFORM: Einzel
      (D) TOPOLOGIE: linear
   (ii) ART DES MOLEKÜLS: Protein
   (v) ART DES FRAGMENTS: inneres
   (xi) SEQUENZBESCHREIBUNG: SEQ ID NO: 1:
   Met Lys Lys Phe Asn Leu Ile Ile Glu Ala Leu Phe Ala Ile
   Leu Leu
   1 5 10
   15
   Thr Ala Cys Asn Phe Gly Leu Met Glu Glu Thr Lys Ile Ala
   Leu Glu
   20 25 30
   Ser Ser Ser Lys Asp Val Lys Asn Lys Ile Leu Gln Ile Lys
   Lys Asp
   35 40 45
   Ala Glu Asp Lys Gly Val Asn Phe Ala Ala Phe Thr Ser Ser
   Glu Thr
   50 55 60
   Gly Ser Lys Val Thr Asn Gly Gly Leu Ala Leu Arg Glu Ala
   Lys Ile
   65 70 75
   80
   Gln Ala Ile Asn Glu Val Glu Lys Phe Leu Lys Arg Ile Glu
   Glu Glu
   85 90
   95
   Ala Leu Lys Leu Lys Glu His Gly Asn Ser Gly Gln Phe Leu
   Glu Leu
   100 105 110
   Phe Asp Leu Leu Leu Glu Val Leu Glu Ser Leu Glu Pro Ile
   Gly Ile
   115 120 125
   Lys Gly Leu Lys Asp Phe Ile Ser Glu Glu Ala Lys Cys Asn
   Pro Ile
   130 135 140
   Ser Thr Ser Glu Arg Leu Ile Glu Val Lys Val Gln Ile Glu
   Asn Lys
   145 150 155
   160
   Met Glu Glu Val Lys Arg Lys Gln Asn Leu Asn Lys Glu Arg
   Lys Ser
   165 170
   175
   Asn Lys Gly Lys Lys Lys Lys
   180
(2) INFORMATION ZU SEQ ID NO: 2:
   (i) SEQUENZ CHARAKTERISTIKA:
      (A) LÄNGE: 170 Aminosäuren
      (B) ART: Aminosäure
      (C) STRANGFORM: Einzel
      (D) TOPOLOGIE: linear
   (ii) ART DES MOLEKÜLS: Protein
   (v) ART DES FRAGMENTS: inneres
   (xi) SEQUENZBESCHREIBUNG: SEQ ID NO: 2:
   Met Ile Lys Tyr Asn Lys Ile Ile Leu Thr Leu Thr Leu Leu
   Ala Ser
   1 5 10
   15
   Leu Leu Ala Ala Cys Ser Leu Thr Gly Lys Ala Arg Leu Glu
   Ser Ser
   20 25 30
   Val Lys Asp Ile Thr Asn Glu Ile Glu Lys Ala Ile Lys Glu
   Ala Glu
   35 40 45
   Asp Ala Gly Val Lys Thr Asp Ala Phe Thr Glu Thr Gln Thr
   Gly Gly
   50 55 60
   Lys Val Ala Gly Pro Lys Ile Arg Ala Ala Lys Ile Arg Val
   Ala Asp
   65 70 75
   80
   Leu Thr Ile Lys Phe Leu Glu Ala Thr Glu Glu Glu Thr Ile
   Thr Phe
   85 90
   95
   Lys Glu Asn Gly Ala Gly Glu Asp Glu Phe Ser Gly Ile Tyr
   Asp Leu
   100 105 110
   Ile Leu Asn Ala Ala Lys Ala Val Glu Lys Ile Gly Met Lys
   Asp Met
   115 120 125
   Thr Lys Thr Val Glu Glu Ala Ala Lys Glu Asn Pro Lys Thr
   Thr Ala
   130 135 140
   Asn Gly Ile Ile Glu Ile Val Lys Val Met Lys Ala Lys Val
   Glu Asn
   145 150 155
   160
   Ile Lys Glu Lys Gln Thr Lys Asn Gln Lys
   165 170
(2) INFORMATION ZU SEQ ID NO: 3:
   (i) SEQUENZ CHARAKTERISTIKA:
      (A) LÄNGE: 552 Basenpaare
      (B) ART: Nukleinsäure
      (C) STRANGFORM: Einzel
      (D) TOPOLOGIE: linear
   (ii) ART DES MOLEKÜLS: Genom-DNA
   (xi) SEQUENZBESCHREIBUNG: SEQ ID NO: 3:
   ATGAAAAAGT TCAATTTAAT AATTGAGGCG CTGTTTGCTA TTCTATTAAC AGCTTGTAAT 60
   TTTGGATTAA TGGAAGAAAC AAAAATAGCG CTTGAATCAT CCTCTAAGGA TGTAAAAAAT 120
   AAAATTTTAC AAATAAAAAA AGACGCTGAG GACAAGGGTG TAAATTTTGC AGCTTTTACA 180
   AGCAGTGAAA CCGGTTCTAA AGTGACAAAT GGAGGATTAG CTTTAAGAGA AGCAAAAATA 240
   CAAGCAATTA ATGAAGTGGA AAAGTTTCTC AAGAGAATAG AAGAAGAGGC TTTAAAACTT 300
   AAAGAACATG GAAATAGTGG TCAATTCTTG GAGCTGTTTG ACTTACTGCT TGAAGTTTTA 360
   GAATCATTAG AACCGATTGG AATAAAAGGC TTAAAAGACT TTATTTCAGA GGAAGCTAAA 420
   TGTAACCCTA TAAGCACATC TGAAAGATTA ATTGAGGTTA AGGTGCAAAT AGAAAATAAG 480
   ATGGAAGAGG TTAAGAGAAA ACAAAATCTT AATAAGGAGA GAAAAAGTAA TAAAGGCAAA 540
   AAAAAGAAAT AA 552
(2) INFORMATION ZU SEQ ID NO: 4:
   (i) SEQUENZ CHARAKTERISTIKA:
      (A) LÄNGE: 513 Basenpaare
      (B) ART: Nukleinsäure
      (C) STRANGFORM: Einzel
      (D) TOPOLOGIE: linear
   (ii) ART DES MOLEKÜLS: Genom-DNA
   (xi) SEQUENZBESCHREIBUNG: SEQ ID NO: 4:
   ATGATTAAAT ATAATAAAAT TATACTTACA CTAACTTTAC TTGCTAGCCT GTTAGCAGCA 60
   TGTAGTTTAA CAGGAAAAGC TAGATTGGAA TCATCAGTTA AAGACATTAC AAATGAAATA 120
   GAGAAAGCTA TAAAAGAAGC TGAAGACGCT GGTGTAAAGA CAGACGCGTT CACAGAAACA 180
   CAAACAGGTG GCAAGGTGGC AGGCCCTAAA ATAAGAGCAG CAAAAATACG CGTCGCTGAC 240
   TTAACAATCA AATTCCTAGA AGCAACAGAA GAGGAAACTA TTACATTTAA AGAAAATGGA 300
   GCGGGGGAAG ATGAATTCTC AGGAATATAC GATTTAATAC TCAACGCCGC AAAAGCAGTA 360
   GAAAAAATTG GGATGAAAGA TATGACAAAA ACGGTCGAAG AGGCCGCTAA AGAAAATCCT 420
   AAAACTACAG CTAATGGGAT AATTGAGATT GTAAAAGTAA TGAAAGCAAA AGTGGAAAAC 480
   ATTAAAGAAA AACAAACTAA AAATCAAAAA TAA 513
(2) INFORMATION ZU SEQ ID NO: 5:
   (i) SEQUENZ CHARAKTERISTIKA:
      (A) LÄNGE: 12 Aminosäuren
      (B) ART: Aminosäure
      (C) STRANGFORM: Einzel
      (D) TOPOLOGIE: linear
   (ii) ART DES MOLEKÜLS: Peptid
   (v) ART DES FRAGMENTS: inneres
   (xi) SEQUENZBESCHREIBUNG: SEQ ID NO: 5:
   Thr Asp Ala Phe Thr Glu Thr Gln Thr Gly Gly Lys
   1 5 10
(2) INFORMATION ZU SEQ ID NO: 6:
   (i) SEQUENZ CHARAKTERISTIKA:
      (A) LÄNGE: 8 Aminosäuren
      (B) ART: Aminosäure
      (C) STRANGFORM: Einzel
      (D) TOPOLOGIE: linear
   (ii) ART DES MOLEKÜLS: Peptid
   (v) ART DES FRAGMENTS: inneres
   (xi) SEQUENZBESCHREIBUNG: SEQ ID NO: 6:
   Asp Ile Thr Asn Glu Ile Glu Lys
    1               5
(2) INFORMATION ZU SEQ ID NO: 7:
   (i) SEQUENZ CHARAKTERISTIKA:
      (A) LÄNGE: 13 Aminosäuren
      (B) ART: Aminosäure
      (C) STRANGFORM: Einzel
      (D) TOPOLOGIE: linear
   (ii) ART DES MOLEKÜLS: Peptid
   (v) ART DES FRAGMENTS: inneres
   (xi) SEQUENZBESCHREIBUNG: SEQ ID NO: 7:
   Phe Leu Glu Ala Thr Glu Glu Glu Thr Ile Thr Phe Lys
    1               5                  10
(2) INFORMATION ZU SEQ ID NO: 11:
   (i) SEQUENZ CHARAKTERISTIKA:
      (A) LÄNGE: 37 Basenpaare
      (B) ART: Nukleinsäure
      (C) STRANGFORM: Einzel
      (D) TOPOLOGIE: linear
   (ii) ART DES MOLEKÜLS: Genom-DNA
   (xi) SEQUENZBESCHREIBUNG: SEQ ID NO: 11:
   GAGGGATCCA TCATGATTAA ATATAATAAA ATTATAC
(2) INFORMATION ZU SEQ ID NO: 12:
   (i) SEQUENZ CHARAKTERISTIKA:
      (A) LÄNGE: 38 Basenpaare
      (B) ART: Nukleinsäure
      (C) STRANGFORM: Einzel
      (D) TOPOLOGIE: linear
   (ii) ART DES MOLEKÜLS: Genom-DNA
   (xi) SEQUENZBESCHREIBUNG: SEQ ID NO: 12:
   GAGGGATCCA TCATGAAAAG TTTAACAGGA AAAGCTAG
(2) INFORMATION ZU SEQ ID NO: 13:
   (i) SEQUENZ CHARAKTERISTIKA:
      (A) LÄNGE: 36 Basenpaare
      (B) ART: Nukleinsäure
      (C) STRANGFORM: Einzel
      (D) TOPOLOGIE: linear
   (ii) ART DES MOLEKÜLS: Genom-DNA
   (xi) SEQUENZBESCHREIBUNG: SEQ ID NO: 13:
   GGACTGCAGG TCGACTTATT TTTGATTTTT AGTTTG

## Beschreibung

Die Lyme-Borreliose ist die häufigste von Zecken übertragene Infektionskrankheit des Menschen. Ein erheblicher Teil der Zecken, die als Vektoren für die Übertragung der Lyme-Borreliose dienen, ist mit dem Erreger der Lyme-Borreliose, der Spirochäte Borrelia burgdorferi, durchseucht. Der Durchseuchungsgrad kann je nach geographischem Gebiet zwischen 1 % und bis zu 100 % liegen.

Eine Infektion mit B.burgdorferi führt zu einem komplexen Krankheitsbild, das man in verschiedene Stadien einteilen kann.

In einigen Fällen kann die Infektion mit B.burgdorferi subklinisch verlaufen. Problematisch sind jedoch häufig Spätfolgen, die durch nicht erkannte bzw. nicht behandelte Borrelien-Infektionen verursacht werden. Insbesondere wegen der gefährlichen Erkrankungen, die bei nicht erkannten bzw. nicht behandelten Infektionen auftreten können, wie Karditis, Myositis, Iritis, Panophthalimitis oder neurologische Manifestationen ist es wichtig, eine etwaige Infektion mit B.burgdorferi möglichst sicher und zutreffend diagnostizieren zu können.

Der Erregernachweis kann insbesondere im Frühstadium aus Patientenmaterial geführt werden. Hierbei ist allerdings nachteilig, daß die Anzüchtung von B.burgdorferi verhältnismäßig schwierig ist und daher in der Regel Speziallabors vorbehalten bleibt.

Wünschenswert ist es daher, die Antikörper aus dem Serum, bei neurologischen Manifestationen auch aus Liquor und bei Gelenkbeschwerden aus Gelenkpunktat nachzuweisen.

Für die Diagnostik ist es wichtig, bestimmen zu können, ob eine Infektion erst vor kurzem stattgefunden hat, oder ob es sich um eine Infektion handelt, die schon einige Zeit zurückliegt. Diese Unterscheidung kann durch die immunologische Bestimmung der nachgewiesenen Antikörper geführt werden, wobei in der Regel IgM-Antikörper für eine erst kürzlich zurückliegende Infektion sprechen, wohingegen IgG-Antikörper für eine bereits länger zurückliegende Infektion sprechen.

Für die Diagnostik ist auch wichtig, daß die diagnostischen Tests spezifisch sind, d.h. daß keine Kreuzreaktionen auftreten mit solchen bakteriellen Erregern, die eine gewisse phylogenetische Verwandtschaft zu den Borrelien aufweisen, wie beispielsweise Treponema pallidum.

Andererseits ist es aber für die Diagnostik auch bedeutsam, daß durch die in den Testverfahren eingesetzten Proteine bzw. Peptide möglichst alle Stämme von Borrelia burgdorferi erkannt werden können.

Da die Lyme-Borreliose weitverbreitet ist und, dä eine Infektion leicht durch einen Zeckenbiß übertragen werden kann, besteht auch ein erhebliches Bedürfnis, Impfstoffe zu entwickeln, die einen Immunschutz gegen Borrelien-Infektionen gewährleisten.

Für die Entwicklung von Impfstoffen eignen sich insbesondere solche Proteine, die an der Oberfläche der Bakterien mit dem Immunsystem des befallenen Organismus in Kontakt kommen.

In WO 96/34106 und WO 97/27301 werden andere Proteine aus Borrelia burgdorferi beschrieben, die dort als "decorin binding proteins" bezeichnet werden.

Im Rahmen der vorliegenden Erfindung konnten nun zwei Proteine aufgefunden werden, die sowohl für die Diagnostik wie auch für die Entwicklung von Impfstoffen besonders geeignet sind.

Gegenstand der vorliegenden Erfindung sind daher immunologisch aktive Proteine von Borrelia burgdorferi, die in einer von anderen aus Borrelia burgdorferi stammenden Proteinen freien Form vorliegen, und die Sequenz des Proteins 1829-22B mit der Aminosäuresequenz (Seq.ID: 2) oder eine Teilsequenz davon mit wenigstens 10 aufeinanderfolgenden Aminosäuren aufweisen. Offenbart wird auch die Sequenz des Proteins 1829-22A mit der Aminosäuresequenz (Seq.ID: 1) oder eine Teilsequenz davon mit wenigstens 10 aufeinanderfolgenden Aminosäuren.

Erfindungsgemäß werden bevorzugt solche Teilsequenzen eingesetzt, die Epitope aufweisen, die diagnostisch und/oder therapeutisch relevant sind. Bei dem Protein 1829-22A, dessen Sequenz im sequenzprotokoll unter Seq.ID No. 1 aufgeführt ist, sind folgende Teilsequenzen besonders bevorzugt:

Der Bereich zwischen der Aminosäure 31 (Lys) und der Aminosäure 55 (Asn). Ein anderes bevorzugtes Polypeptid befindet sich zwischen Position 60 (Thr) und Position 71 (Gly). Ein weiteres bevorzugtes Polypeptid befindet sich zwischen Aminosäure 82 (Gln) und Aminosäure 108 (Gln). Weiterhin ist besonders bevorzugt der C-terminale Bereich zwischen Aminosäure 130 (Gly) und Aminosäure 183 (Lys).

Bei dem Protein 1829-22B, das mit der Seq.ID No. 2 wiedergegeben ist, sind folgende Teilbereiche besonders bevorzugt: Aminosäure 61 (Gln) bis Aminosäure 71 (Ile); Aminosäure 87 (Glu) bis Aminosäure 108 (Gly); Aminosäure 121 (Glu) bis Aminosäure 145 (Asn) und der C-terminale Bereich von Aminosäure 150 (Ile) bis Aminosäure 170 (Lys). Die Positionen der Aminosäuren sind in den Sequenzprotokollen angegeben. Die Peptide, die die oben angegebenen Teilsequenzen aufweisen, können entweder durch chemische Synthese hergestellt werden oder gentechnologisch in geeigneten Wirtssystemen exprimiert werden.

Die erfindungsgemäßen Proteine bzw. Peptide können mit Hilfe von gentechnologischen Methoden hergestellt werden, was den Vorteil hat, daß keine anderen von B.burgdorferi herstammenden Proteine mit den gewünschten Proteinen assoziiert sind. Alternativ können geeignete Peptide auch auf klassische chemische Art und Weise synthetisiert werden; derartige Peptide sind auch frei von immunologisch inaktiven Verunreinigungen. Es ist jedoch durchaus möglich, die erfindungsgemäßen Proteine bzw. Peptide zusammen mit anderen isolierten Proteinen von B.burgdorferi in Testkits oder bei Impfstoffen einzusetzen.

Der im Rahmen der vorliegenden Erfindung verwendete Begriff "immunologisch aktives Protein" umfaßt nicht nur ein Protein, das die vollständige Aminosäuresequenz des Proteins 1829-22B umfaßt, sondern auch Teile dieses Proteins, die wenigstens so lang sind, daß sie mindestens ein lineares Epitop umfassen. Im allgemeinen beträgt die Mindestlänge eines erfindungsgemäßen Peptids, das eine Epitopeigenschaft aufweisen kann, wenigstens sechs, bevorzugt 10, besonders bevorzugt 25 und ganz besonders bevorzugt wenigstens 50 Aminosäuren.

Es muß berücksichtigt werden, daß bei den einzelnen Stämmen von Borrelia burgdorferi zumindest geringfügige Veränderungen in der Aminosäuresequenz des Proteins in Abhängigkeit von dem jeweiligen Stamm auftreten. Daher betrifft die vorliegende Erfindung auch solche immunologisch aktiven Proteine bzw. Peptide, die eine hohe Homologie zu den oben beschriebenen Aminosäuresequenzen aufweisen.

Die erfindungsgemäßen immunologisch aktiven Proteine bzw. Peptide weisen eine Homologie von wenigstens 60 %, bevorzugt wenigstens 80 % und besonders bevorzugt von wenigstens 90 % bezogen auf das erfindungsgemäße Protein 1829-22B auf. Unter dem Begriff einer Homologie von 90 % versteht man beispielsweise, daß in dem homologen Peptid neun von 10 Aminosäuren identisch sind mit den entsprechenden Aminosäuren an der homologen Stelle in der Aminosäuresequenz 1829-22B.

Im Rahmen der vorliegenden Erfindung sind solche Bereiche der erfindungsgemäßen Proteine bzw. Peptide besonders wichtig, die Epitope aufweisen, also solche Stellen im Protein, an die Antikörper spezifisch binden. Die Bestimmung, an welchen Stellen Epitope zu erwarten sind, kann entweder durch an sich bekannte Computermethoden festgelegt werden oder es ist auch möglich, bestimmte kurze Peptide mit einer Länge von wenigstens 10, bevorzugt wenigstens 25 Aminosäuren zu synthetisieren. Diese Peptide werden dann mit Hilfe von positiven Seren getestet, und zwar, ob immunologische Reaktionen stattfinden oder nicht. Auf diese Art und Weise können lineare Epitope bestimmt werden. Bei der Herstellung dieser Proteine bzw. Peptide können entweder gentechnologische Verfahren verwendet werden, wobei die Peptide beispielsweise als Fusionsproteine in Mikroorganismen exprimiert werden oder die Peptide können mit Hilfe der klassischen Synthese (Merrifield-Technik) synthetisiert werden.

Die Bestimmung von immunologisch relevanten Epitopen ist nicht nur für die Diagnostik, sondern insbesondere auch für die Herstellung von Impfstoffen wichtig. Für Impfstoffe können immunologisch sehr reaktive Bereiche aus den erfindungsgemäßen Proteinen kombiniert werden mit entsprechenden Bereichen aus anderen, bereits bekannten Proteinen von Borrelia burgdorferi, wie OspA, OspC oder mit Aminosäuresequenzen aus dem Flagellin.

Gegenstand der vorliegenden Erfindung sind auch Testkits zum Nachweis von Antikörpern gegen Borrelia-Stämme, die wenigstens ein erfindungsgemäßes immunologisch aktives Protein enthalten, das mit den in der Untersuchungsflüssigkeit vorhandenen Antikörpern reagieren kann und die wenigstens eine Anzeigekomponente aufweisen, die den Nachweis von Komplexen aus immunologisch aktivem Protein und Antikörper ermöglichen.

Bevorzugt sind Testkits, die wenigstens ein immunologisch aktives Protein mit einer Teilsequenz des Proteins 1829-22A und wenigstens ein Protein mit einer Teilsequenz des Proteins 1829-22B aufweisen.

Bei der Anzeigekomponente kann es sich um einen gegen den nachzuweisenden Antikörper gerichteten Antikörper handeln, der eine Markierung aufweist. Bevorzugt handelt es sich hierbei um einen Antihuman-IgG- oder Antihuman-IgM-Antikörper. Bei der Markierung handelt es sich häufig um ein Enzym, das eine Farbreaktion katalysieren kann.

Eine Möglichkeit des Nachweises besteht darin, daß das erfindungsgemäße immunologisch aktive Protein oder ein dagegen gerichteter monoklonaler Antikörper biotinyliert ist und, daß die Anzeigekomponente Avidin oder Streptavidin mit daran kovalent gebundenem Enzym, insbesondere Peroxidase ist.

In einer bevorzugten Ausführungsform der Erfindung handelt es sich bei dem Testkit um ein ELISA-Testkit. Bei einer besonders bevorzugten Ausführungsform der vorliegenden Erfindung ist wenigstens ein erfindungsgemäßes immunologisch aktives Protein an Mikrotiterplatten gekoppelt und die Anzeigekomponente besteht aus Anti-Human-Immunoglobulin, insbesondere IgG- und/oder IgM-Antikörpern, an die ein eine Farbreaktion katalysierendes Enzym gekoppelt ist.

In einer weiteren bevorzugten Ausführungsform der vorliegenden Erfindung handelt es sich bei dem Testkit um einen Immun-Blot, der auch als Protein-Blot oder Western-Blot bezeichnet wird. Bei derartigen Testkits wird Protein mit Hilfe eines Elektrophoresegels, beispielsweise eines Polyacrylamidgels auf eine immobilisierende Matrix (z.B. Nitrocellulose-Filter) über-tragen. Die Übertragung kann beispielsweise durch Elektro-transfer erfolgen. Anschließend findet eine immunologische Reaktion zwischen den auf der Matrix befindlichen Proteinen und den gegen die Proteine gerichteten Antikörpern statt. Die Antikörper können dann durch geeignete Methoden nachgewiesen werden, z.B. durch enzymmarkierte Anti-Antikörper.

Unter dem Begriff "Testkits" wird ein Satz von Testreagenzien verstanden, der den Nachweis von bestimmten Antikörpern ermöglicht. Die erfindungsgemäßen Testkits weisen als erfindungsgemäße Komponente wenigstens ein erfindungsgemäßes protein bzw. Peptid auf. Das immunologisch aktive Protein bzw. Peptid wirkt als Antigen und reagiert mit den in der Untersuchungsflüssigkeit vorhandenen Antikörpern. Die erfindungsgemäßen Testkits können auf verschiedenen, an sich bekannten Prinzipien beruhen. In der Regel findet eine Reaktion zwischen dem Antigen und Antikörpern statt und diese Reaktion bzw. der hierbei entstandene Komplex wird nachgewiesen. Es ist möglich, daß das Antigen an eine feste Phase, wie eine Mikrotiterplatte oder magnetische Kügelchen gebunden ist. Dieses Antigen kann dann mit der Untersuchungsflüssigkeit (Serum oder Liquor) in Kontakt gebracht werden. Dabei binden die in der Untersuchungsflüssigkeit vorhandenen Antikörper an das Antigen. Üblicherweise wird dann gewaschen und die gebundenen Antikörper werden nachgewiesen durch Anti-Antikörper, die eine Markierung tragen. Bei der Markierung kann es sich um ein radioaktives Isotop handeln oder ein Enzym, das eine Farbreaktion katalysiert, wie beispielsweise die Meerrettich-Peroxidase.

Es gibt jedoch eine Vielzahl von Testausgestaltungen, die dem Fachmann an sich bekannt sind. So kann beispielsweise auch der Anti-Antikörper an eine feste Phase gebunden sein und das Antigen kann eine nachweisbare Markierung aufweisen.

Im Rahmen der vorliegenden Erfindung sind insbesondere solche Testkits bevorzugt, die zur Durchführung eines ELISA (Enzym-Linked Immunosorbent Assay) oder zur Durchführung eines sogenannten Western blots geeignet sind.

Da die Verwendung von radioaktiv markierten Markierungssubstanzen mehr und mehr auf Widerstand stößt, wird erfindungsgemäß bevorzugt der Komplex bestehend aus Antigen/nachzuweisendem Antikörper und Anti-Antikörper dadurch nachgewiesen, daß entweder Antigen oder Anti-Antikörper biotinyliert sind. Der Nachweis des Komplexes erfolgt dann durch Zugabe von Avidin, an das beispielsweise ein eine Farbreaktion katalysierendes Enzym gekoppelt ist.

Im Rahmen der vorliegenden Erfindung ist auch der sogenannte µ-Capture-Test besonders bevorzugt. Bei dem µ-Capture-Test wird an die Festphase ein Antikörper gegen humane IgM-Anti-körper (µ-Ketten) gebunden. Diese Anti-Antikörper fangen aus dem Serumgemisch sowohl für das Antigen spezifische, wie auch unspezifische IgM-Antikörper heraus. Nach Zugabe von Antigen, welches direkt markiert sein kann, wird die IgM-Immunantwort nachgewiesen. Alternativ hierzu kann auch nicht-markiertes Antigen eingesetzt werden und das Antigen (erfindungsgemäßes immunologisch aktives Protein) wird durch einen weiteren markierten Antikörper gegen das Antigen nachgewiesen. Bei der Markierung kann es sich beispielsweise um ein Enzym handeln, das eine Farbreaktion katalysiert.

Die erfindungsgemäßen immunologisch aktiven Proteine bzw. Peptide können auch zur Herstellung von monoklonalen Antikörpern verwendet werden. Die Herstellung von monoklonalen Antikörpern erfolgt nach an sich bekannten Standardverfahren.

Gegenstand der vorliegenden Erfindung sind weiterhin Impfstoffe, die wenigstens ein erfindungsgemäßes Protein bzw. Peptid enthalten. Die erfindungsgemäßen immunologisch aktiven Proteine von Borrelia burgdorferi können also zur Herstellung eines Impfstoffs zum Schutz gegen Infektionen von Borrelia burgdorferi-Bakterien verwendet werden.

Für die Herstellung eines Impfstoffes ist es wesentlich, diejenigen Bereiche in immunologisch aktiven Proteinen festzustellen, die die Bildung von schützenden Antikörpern hervorrufen. Wenn die immunologisch aktiven Proteine dem zu impfenden Organismus appliziert werden, müssen sich solche Antikörper bilden, die bei einer Infektion von Borrelia burgdorferi an die eingedrungenen Bakterien binden und, die die Abtötung der eingedrungenen Bakterien durch das körpereigene Immunsystem ermöglichen. Die erfindungsgemäßen Impfstoffe werden bevorzugt zur Impfung von Menschen, aber auch zur Impfung von Tieren verwendet. Eine Impfung ist vor allem sinnvoll für Tiere, die durch Zecken gebissen werden können und dadurch von Borrelia burgdorferi infiziert werden können. Insbesondere ist eine Impfung sinnvoll bei Hunden oder Pferden.

Gegenstand der vorliegenden Erfindung sind auch Nukleinsäuren, die für das erfindungsgemäße immunologisch aktive Protein kodieren.

Bevorzugt handelt es sich hierbei um eine Nukleinsäure, die eine DNA-Sequenz aufweist, die für das Protein 1829-22B kodiert und die Sequenz (Seq.ID: 4) besitzt oder eine Teilsequenz davon, die wenigstens 18 Nukleotide umfaßt.

Erfindungsgemäß sind auch Teilsequenzen der oben genannten Sequenzen bevorzugt, die wenigstens 30 und besonders bevorzugt 50 Nukleotide aufweisen.

Die erfindungsgemäßen Nukleinsäuren, Nukleinsäurefragmente sowie damit hybridisierende Nukleinsäurefragmente mit einer Länge von mindestens 12 Nukleotiden können zum Nachweis einer Infektion von Borrelia burgdorferi mit Hilfe der Polymerase Kettenreaktion eingesetzt werden.

Bei den erfindungsgemäßen Nukleinsäuren handelt es sich bevorzugt um DNA-Sequenzen. Die erfindungsgemäßen DNA-Sequenzen sind erforderlich, um die erfindungsgemäßen immunologisch aktive Proteine von Borrelia burgdorferi mit Hilfe von gentechnologischen Methoden herzustellen. Es ist aber auch besonders vorteilhaft, Teilsequenzen der erfindungsgemäßen Sequenzen für diagnostische Verfahren einzusetzen, wobei das PCR-Verfahren eine weitgehende Verbreitung gefunden hat. Hierzu werden kurze Fragmente der erfindungsgemäßen Nukleinsäuren synthetisiert, die mit den komplementären Sequenzen in der Untersuchungsprobe hybridisieren können. Durch die Polymerase Kettenreaktion (PCR) werden dann geringste Mengen der gesuchten Nukleinsäuren amplifiziert und anschließend nachgewiesen.

Eine andere bevorzugte Verwendung der erfindungsgemäßen Nukleinsäuren ist die DNA-Vaccinierung. Dabei werden die erfindungsgemäßen Nukleinsäuren bzw. Teile davon in den zu immunisierenden Wirt eingebracht, wobei die Nukleinsäure entweder in nackter Form oder in Form von Plasmiden oder retroviralen Vektoren vorliegen kann. Die DNA wird dann im Wirtsorganismus translatiert und durch die translatierten Genprodukte erfolgt eine Immunisierung im Wirt.

Die vorliegende Erfindung wird durch die nachfolgenden Beispiele weiter erläutert.

### Beispiel 1

### Bestimmung von Partialsequenzen

Aus Lysaten von B. burgdorferi, Stamm Pko, wurde durch Extraktion mit n-Octyl β-D-Thioglucopyranosid eine darin lösliche Proteinfraktion partiell aufgereinigt und weiter durch SDS-Polyacrylamid-Gel-Elektrophorese aufgetrennt. Anschließend wurden aus dem niederen Molekulargewichtsbereich (<30kDa) Antigene durch Western Blotting auf eine Glasfibermatrix übertragen und die entsprechenden Stücke mit B. burgdorferi Antigenen ausgeschnitten (nach Eckerskorn et al., 1988, Eur.J.Biochem. 176: 509 - 519, A new siliconized fiber as support for protein-chemical analysis of electroblotted proteins.). Eines dieser so erhaltenen Proteine wurde näher untersucht. Eine direkte Ansequenzierung war nicht möglich, da der N-Terminus einer Sequenzierung nicht zugänglich war. Das Antigen wurde deshalb im Gel gespalten und anschließend wurden die Peptide extrahiert.

Dazu wurde das Antigen mittels SDS-PAGE aufgetrennt und das Gel anschließend mit Coomassie-Blau angefärbt. Nach Lokalisation der Proteinbanden wurde das Gel mit 10 % Essigsäure entfärbt. Die Proteinbande und eine entsprechende Referenzbande ohne Antigen, mit der gleichen Größe, wurde ausgeschnitten, durch ein Sieb gedrückt und zerkleinert (Poren 30 µm x 100 µm). Das zerquetschte Gel wurde mit ½ konz. Inkubationspuffer 2 Min gewaschen (12.5 M Tris, 0.5 M EDTA, pH 8,5), abzentrifugiert und der Puffer entfernt. Das so extrahierte Gel wurde 1h in der Vakuum-Zentrifuge angetrocknet bis zu einem Restwasser-Gehalt von etwa 5 % und einer gummiartigen Konsistenz. Endoprotease LysC wurde in 400 µl 12,5 M Tris/HCl, pH 8,5 gelöst (Enzym : Protein = 1:10) und 0.1 % Laurylmaltosit zugefügt. Je 200 µl wurden zu Probe und Referenz gegeben und über Nacht bei 37°C im Heizblockschüttler inkubiert. Danach wurden die Peptide mittels reversed phase Chromatographie auf einer HPLC-Anlage aufgetrennt, und die Sequenz ausgesuchter Peptide analysiert. Der N-terminale Abbau der Aminosäuren und damit die Sequenzbestimmung erfolgte in einem automatischen Sequenzer Porton 3600 (Beckmann Instruments) nach Edman & Begg (1967, Eur.J.Biochem. 1, 80-91). Hierbei wurden folgende Peptide nach Sequenzierung erhalten:

### Beispiel 2

### Bereitstellung der Sonden

Aus den gemäß Beispiel 1 gewonnenen Aminosäure-Abfolgen wurden die nachfolgend aufgeführten Oligodesoxynukleotid-Sequenzen abgeleitet. Da meist mehrere Codon-Möglichkeiten für eine Aminosäure bestehen, mußten an den entsprechenden Stellen des Oligonukleotids auch Basenvariationen berücksichtigt werden und während der Synthese in äquimolaren Verhältnissen eingebaut werden. Zur Vermeidung von zu großen Basenvariationen wurde die Häufigkeit der Kcdons von bereits bekannten und molekularbiologisch charakterisierten Gensequenzen, wie z.B. OspA, OspC und p100 analysiert und zur Darstellung der abgeleiteten Oligonukleotidsequenzen verwendet.

### AA1829-22B - Oligodesoxynukleotid-Sequenzen

Die in Klammern angegebenen und durch ";" getrennten Basen wurden während der Synthese (auf Pharmacia DNA Synthesizer) in äquimolaren Verhältnissen eingebaut:

### Beispiel 3

### Auffinden des kodierenden Genfragmentes

Die Oligonukleotid-Sequenzen wurden als Sonden verwendet und im Southern Blot nach Markierung mit Digoxigenin eingesetzt (nach The DIG system User's Guide for Filter Hybridization, Boehringer Mannheim GmbH). Dazu wurde chromosomale DNA vom Borrelia burgdorferi Stamm Pko nach üblicher Methode (siehe Maniatis: Molecular Cloning, A laboratory Manual) isoliert, mit verschiedenen Restriktionsendonukleasen enzymatisch ge-spalten und in 1 % Agarose-Gel aufgetrennt. Die DNA aus den Agarose-Gelen wurde nach vorheriger Denaturierung auf Nylonmembranen übertragen (Southern Blotting: nach The DIG system User's Guide for Filter Hybridization, Boehringer Mannheim GmbH). Die Hybridisierung wurde nach Standardbedingungen (s.o. Boehringer Mannheim GmbH) durchgeführt. Gebundene Oligodesoxynukleotide wurden mittels anti-Diooxigenin Antikörpern, an die alkalische Phosphatase gekoppelt sind, immunologisch nachgewiesen. Entsprechende hybridisierende Banden wurden nach Anfärbung identifiziert. Solche identifizierte Banden wurden nach präparativer Agarose-Gelelektrophorese aus dem Gel eluiert, durch EtOH gefällt, in geeignetem Puffer aufgenommen und mit E.coli Plasmiden ligiert, die mit entsprechenden Restriktionsendonukleasen enzymatisch gespaltenen wurden. Die so ligierte DNA wurde in kompetente E. coli Zellen eingeschleust und transformierte E. coli Zellen wurden erhalten. Die positiven E. coli Zellen wurden von den negativen Zellen nach Plasmid Screening und Restriktionsendonuklease Spaltung der erhaltenen DNA und anschließend erneuter Hybridisierung mit den Digoxigenin (Boehringer Mannheim) markierten Primern (siehe oben) getrennt. Geeignete Klone wurden durch Hybridisierung aufgefunden. Unter Verwendung von Restrikionsendonuklease mit bevorzugter Erkennung von Hexamer Sequenzen wurde eine Restriktionskarte eines HindIII-HindIII DNA-Inserts erstellt. Darauf erfolgte eine Subklonierung verschiedener DNA Fragmente und daran anschließende Sequenzierung durch automatische Sequenzierung nach Fluoreszenzlabelling (Applied Biosystems, ABI-Prism 377, Weiterstadt).

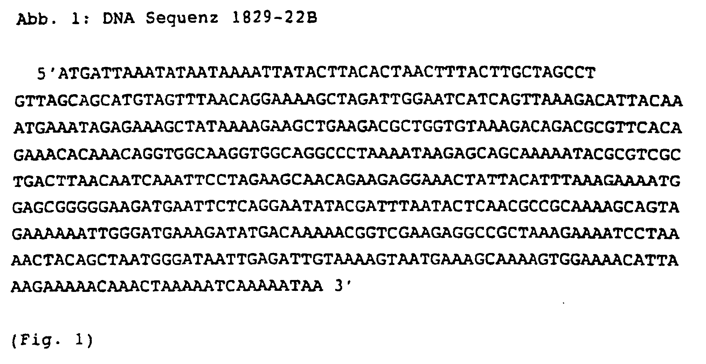

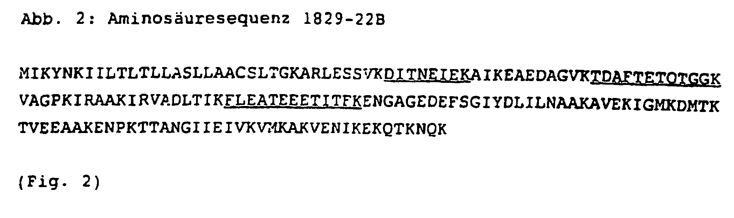

Nach Analyse der so erhaltenen Sequenz mit einem Computerprogramm zur DNA Analyse (DNASTAR, Lasergene London) wurden mehrere offene Leserahmen identifiziert. Wovon 2 Leserahmen typische Signalsequenzen, wie Start des Leserahmens mit Methionin oder Ribosomenbindungstelle in entsprechendem Abstand vor dem Leserahmen aufwiesen. Ein Leserahmen wies deutliche Sequenzübereinstimmung mit den ursprünglich erhaltenen Peptidsequnzen und den davon abgeleiteten Primern auf. Dieser Leserahmen weist die in Figur 1 dargestellte Sequenz auf. Die davon abgeleitete Aminosäuresequenz ist in Figur 2 dargestellt.

Das Protein 1829-22B weist eine Länge von 170 Aminosäuren auf. Die entsprechenden Peptid Sequenzen, die bei der primären Charakterisierung gefunden wurden, sind in Figur 2 unterstrichen.

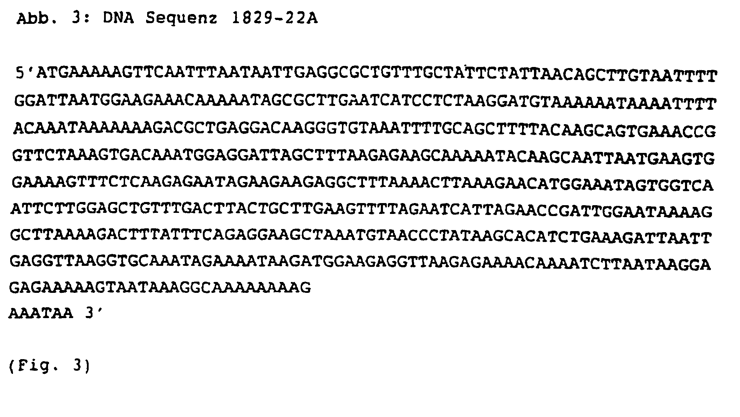

Überraschenderweise wurde auf dem wie oben beschriebenen DNA-Fragment ein weiteres Gen gefunden. Der Leserahmen wurde mit 1829-22A bezeichnet. Die DNA-Sequenz ist in Figur 3 dargestellt.

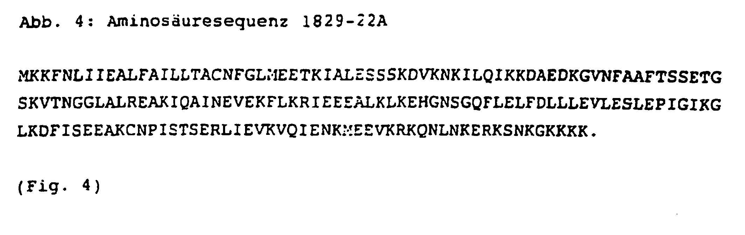

Das davon abgeleitete Protein 1829-22A weist bei einer Länge von 183 Aminosäuren die in Figur 4 dargestellte Sequenz auf.

### Beispiel 4

### Herstellung von Expressionsklonen für das Antigen 1829-22B

Ausgehend von dem kompletten Leserahmen für das Gen 1829-22B wurden Expressionsklone hergestellt. Dabei wurden sowohl der gesamte Leserahmen als auch ein verkürztes Fragment dargestellt. Nachfolgende Oligonukleotid-Primer wurden zur Darstellung der Sequenzen verwendet.

Dabei wurden die Primer-Kombinationen Primer 1 und Primer 3 bzw. Primer 2 und Primer 3 zur Darstellung der vollständigen Sequenz bzw. zur verkürzten Sequenz eingesetzt. Als Template DNA wurde gereinigte DNA von einem das gesamte Insert (1829-22B, 1829-22A und flankierende Sequenzen, HindIII-HindIII-Fragment) enthaltenden Klon verwendet. Die Polymerasekettenreaktion (PCR) wurde nach Herscellervorschrift mit dem PCR-Core Kit (Boehringer Mannheim) durchgeführt. Die Reaktion wurde in einem Thermocycler mit folgendem Programm durchgeführt:

### Programm:

A. Denaturierungszeit 94°C 4min.
B. 35 Zyklen
   "Zyklus: 1.94°C 1min. 2.42°C 1min. 3.72°C 1,5min."
C. Elongation:
   72°C 5min.

Durch die Verwendung von Erkennungssequenzen für Restriktionsendonuklease innnerhalb der Primer Sequenzen konnten so die Amplifikate enzymatisch gespalten werden und in E. coli Plasmid, z.B. pUC8, ebenfalls mit denselben Restriktionsendonukleasen gespalten, ligiert werden. Nach Transformation und Analyse der Klone sowohl durch Agarose-Gelelektrophorese enzymatisch gespaltener DNA, aber auch durch SDS-Polacrylamid-Gelelektrophorese und Coomassie-Blau-Färbüng bzw. anschließendem Transfer auf Nitrocellulose und immunologischer Detektion, wurden positive Klone identifiziert. Dabei stellte sich heraus, daß das verkürzte Fragment deutlich besser darzustellen war und damit auch eine bessere Reaktivität im Westernblot zu erreichen war.

### Beispiel 5

### Reinigung von rekombinanten Borrelia burgdorferi Antigen 1829-22B aus E.coli

Ein Klon, der das Antigen 1829-22B enthält, (pMS1829-22B) wird in 100 ml L-Broth (mit 50 µl Ampicillin/ml) angeimpft, über Nacht wachsen gelassen und dann in 900 ml L-Broth/Ampicillin - doppelt konzentrierter Hefeextrakt/ 2 ml Glycerin - überführt, nach ca. 1h mit 2 mM IPTG induziert und 2- 3h weiter geschüttelt.

Nach Abzentrifugieren bei 8000 Upm für 10 Min. wird das Pellet in 20 ml Lyse Puffer (50 mM Tris-HCl, pH 7,5, 2 mM EDTA, 0,2 mM DTE, 0,1 mM PMSF; 0,4 mg/ml Lysozym) resuspendiert. Nach 30 Min. Rühren bei Raumtemperatur erfolgt Zugabe von Triton-X 100 (Endkonzentration 0,1 - 0,2 %). Zusätzlich werden 10 µl Benzonase (Merck) zugegeben. Es wird weitere 30 Min. bei Raumtemperatur gerührt. Die jetzt klare Suspension wird mit festen NaCl auf 1 M NaCl eingestellt und weitere 30 Minuten bei 4°C gerührt.

Nach Zentrifugation bei 4°C, 30 Minuten, 15.000 Upm befindet sich das Antigen 1829-22B quantitativ im Überstand. Das Pellet wird verworfen. Der Überstand wird gegen 10 mM Tris-HCl, pH 7,0 und 2 mM EDTA bei mehrmaligen Pufferwechsel dialysiert. Nach Zentrifugation und/oder Filtration wird auf DEAE Sepharose (Pharmacia) aufgetragen, wobei die Säule mit 50 mM Tris-HCl und 2 mM EDTA, pH 7,0 equilibriert wird. Bei Elution mit 0 M NaCl befindet sich das Antigen im Durchlauf. Die ersten Fraktionen können verworfen werden, der Rest wird gesammelt und im Dialyseschlauch gegen 50 mM MES (2-[N-Morpholino]ethansulfonsäure) Puffer, pH 6,0 dialysiert. Nach Zentrifugation und Filtration wird das Antigen auf S-Sepahrose fast flow (Pharmacia) Säule aufgetragen. Zuerst wird mit 0 m NaCl gewaschen, dann mit einem Gradienten von 0 - 1 M NaCl eluiert. Das Antigen 1829-22B eluiert als scharfer Peak bei etwa 0,2 M NaCl. Nach Dialyse gegen 10 mM Tris-HCl pH 7,5, kann das Antigen in einem geeigneten Testkit, bspw. einem ELISA oder Westernblot verwendet werden.

### Beispiel 6

### Verwendung von rekombinant produziertem B. burgdorferi Antigen 1829-22B im Westernblot

Gereinigtes Antigen 1829-22B wird dazu in der SDS-Polyacrylamidegelelektrophorese aufgetrennt und auf Nitrozellulose transferiert. Dazu werden SDS-Polyacrylamidgele wie folgt hergestellt. Die SDS-Gele bestehen aus einem Sammelgel und einem Trenngel (nach Laemmli, U.K. 1970, Cleavage of structural proteins during assembly of the head of bacteriphage T4, Nature 227, 680-685). Die Zusammensetzung der Trenngele ist folgendermaßen: 15 % Acrylamid (Bio-Rad), 0,026 % Diallyltartamid (DATD, Bio-Rad) pro Prozent Acrylamid, 0,15 % SDS, 375 mM Tris-HCl pH 8,5, 0,14 mM Ammoniumperoxiddisulphat (AMPER, Bio-Rad) und 0,035 % N, N, N', N'-Tetramethylethylendiamin (TEMED, Bio-Rad). Amper und TEMED dienten dabei als Radikalstarter für die Polymerisation. 2-4 Stunden nach Polymerisation wurde das Sammelgel (3,1 % Acrylamid, 0,08 % DATD, 0,1 % SDS, 125 mM Tris-HCl pH 7,0, 3 mM Amper und 0,05 % TEMED) über das Trenngel gegossen. Die Anoden- und Kathodenkammer wurde mit identischer Pufferlösung gefüllt: 25 mM Tris-Base, 192 mM Glycin und 0,1 % SDS, pH 8,5. Die Antigen enthaltende Probe wurde mit gleichem Teil Probenauftragspuffer (3 % Sacharose, 2 % SDS, 5 % Mercaptoethanol 20 mM Tris-HCl pH 7,0, Bromphenolblau) versetzt, für genau 5 Minuten bei 100°C erhitzt und auf das Sammelgel aufgetragen. Die Elektrophorese wurde bei Raumtemperatur über Nacht mit konstanter Stromstärke von 6 mA für Gele der Größe 20x15 cm durchgeführt. Anschließend wurden die Antigene auf Nitrozellulose (Schleicher und Schuell, Dassel) transferiert.

Für den Proteintransfer befand sich das Gel mit der anliegenden Nitrozellulose zwischen Whatmann 3 MM Filterpapier, leitfähigem, 1 cm dicken Schaumstoff und zwei Kohleplatten, die über Platinelektroden den Strom leiteten. Filterpapier, Schaumstoff und Nitrozellulose wurden gut mit Blot-Puffer (192 mM Glycin, 25 mM Tris-Base, 20 % Methanol, pH 8,5) getränkt. Der Transfer fand bei 2 mA/cm² für 2h statt. Freie Bindungstellen auf der Nitrozellulose wurden für 1h bei 37°C mit Cohen-Puffer (1 mg/ml Ficoll 400, 1 mg/ml Polyvinylpyrrolidon, 16 mg/ml Rinderserumalbumin, 0,1 % NP40, 0,05 % Bacto-Gelatine in Natriumboratpuffer pH 8,2); (nach Cohen et al.: Localisation and Synthesis of an antigenic determinant of Herpes simplex virus glycoprotein D that stimulates the production of neutralizing antibodies. J.Virol. 49, 1984, 4183-4187) abgesättigt. Die Blotstreifen wurden über Nacht bei Raumtemperatur mit Patientenseren (Verdünnung 1:100 in 154 mM NaCl und 10 mM Tris-HCl pH 7,5) inkubiert.

Nach der Seruminkubation wurde der Blot mit TTBS (50 mM Tris-HCl pH 7,5, 500 mM NaCl, 0,01 % Tween 20) viermal für je 15 Minuten gewaschen. Anschließend wurden die Blotstreifen mit Peroxidase-gekoppelten anti-human-IgG Immunglobulin (DAKO, Verdünnung 1:1000 in 154 mM NaCl und 10 mM Tris-HCl, pH 7,5) bzw. anti-human-IgM Immunglobulin (DAKO, Verdünnung 1:500 in 154 mM NaCl und 10 mM Tris-HCl, pH 7,5) für 2h bei Raumtemperatur inkubiert. Nach mehrmaligem Waschen mit TTBS wurden die Blotstreifen mit 10 mg /50 ml Diaminobenzidin und 0,01 % Wasserstoffperoxid in 50 mM Tris-HCl pH 7,5 gefärbt. Die Färbung wurde mit 1N Schwefelsäure gestoppt, der Blot mit Wasser säurefrei gewaschen und zwischen Filterpapier getrocknet.

Die Ergebnisse mit charakterisierten Seren sind in der Tabelle 1 zusammengefaßt:

**Tabelle 1**

| Seren | IgG-reaktiv | IgM reaktiv |
|---|---|---|
| Blutspender n=94 | 0/94 | nicht bestimmt |
| | | |
| Blutspender n=20 | 0/20 | 0/20 |
| | | |
| Lyme Borreliose Patienten (Stadium 2/3) bzw. IgG-Serologie positiv n=24 | 24/24 | nicht bestimmt |
| | | |
| Lyme Borreliose Patienten (Stadium 1) bzw. IgM-Serologie positiv n=10 | nicht bestimmt | 1/10 |

### Beispiel 7

### Nachweis der diagnostischen Bedeutung des rekombinanten Antigens 1829-22B

Für den ELISA wurden rekombinante Borrelia burgdorferi Antigene, wie p100, OspC oder p41/internes Fragment, ohne- oder in Kombination mit gereinigtem p1829-22B-Antigen auf Polystyrolplatten in Carbonat-Puffer, pH 10,6 über Nacht bei 4°C beschichtet. Die Mikrotiterplatten wurden mit BSA-Lösung abgesättigt. Die seruminkubation erfolgte in PBS, 1 % Rinderserumalbumin, pH 7,0 (Verdünnungspuffer) für eine Stunde bei 37°C in der Verdünnung 1+100. Nach viermaligem Waschen mit PBS, 0,05 % Tween 20, pH 7,0 (Waschpuffer) wurde antihuman IgG Peroxidase-Konjugat in Verdünnungspuffer für 30 Min bei 37°C zugegeben. Nach erneutem viermaligen Waschen mit Waschpuffer wurde Substrat TMB zugegeben und für 30 Min im Dunklen bei Raumtemperatur inkubiert. Die Reaktion wird mit Schwefelsäure gestoppt und die Immunfärbung bei 450 nm im Photometer vermessen. Cutoff wurde durch Vermessen von definierten positiven Seren und negativen Seren eingestellt.

Die bei dem Versuch erhaltenen Ergebnisse werden in der nachfolgenden Tabelle 2 zusammengefaßt. Die Ergebnisse zeigen, daß durch Verwendung des erfindungsgemäßen Antigens p1829-22B eindeutigere Ergebnisse erhalten werden konnten. Besonders hervorzuheben ist, daß beim EIA (Enzyme Immuno Assay) ohne p1829-22B mehrere Seren als negativ klassifiziert wurden, die sich im Bestätigungstest mit Hilfe des Immunoblots als positiv herausstellten.

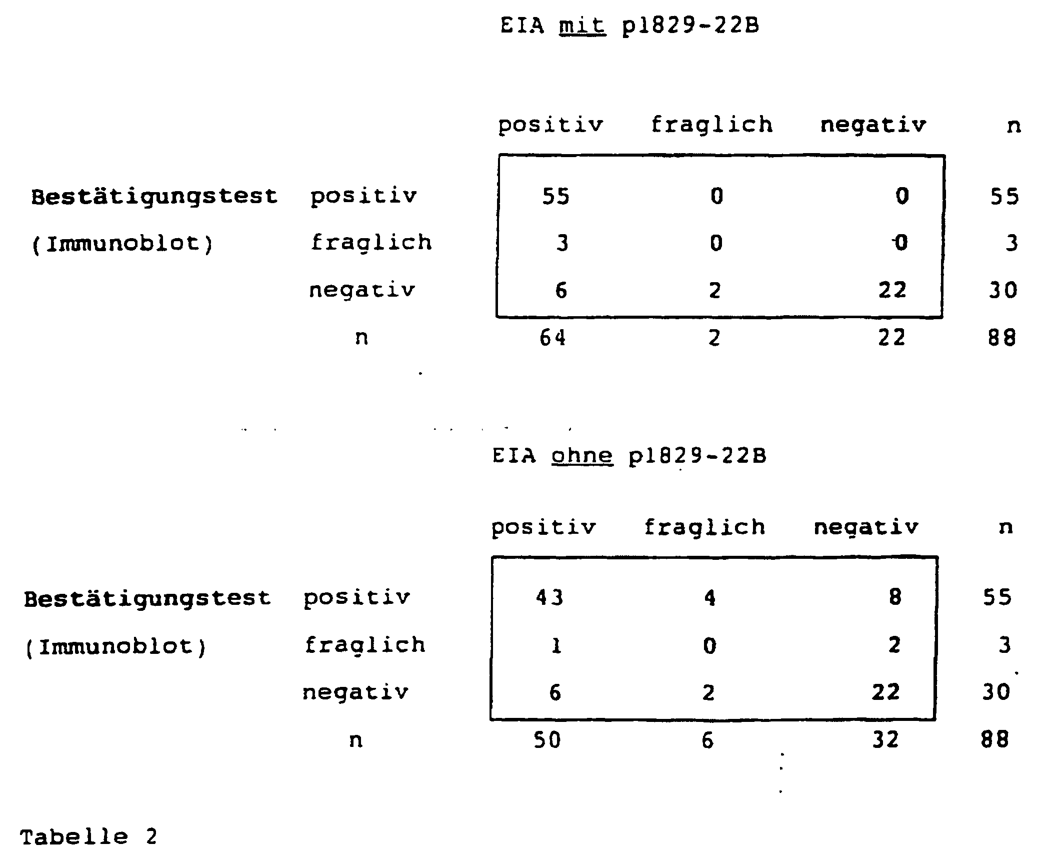

## Patentansprüche

1. Immunologisch aktives Protein von Borrelia burgdorferi, das in einer von anderen aus Borrelia burgdorferi stammenden Proteinen freien Form vorliegt, **dadurch gekennzeichnet, daß** es die Sequenz des Proteins 1829-22B mit der Aminosäuresequenz oder eine Teilsequenz davon mit wenigstens 10 aufeinanderfolgenden Aminosäuren aufweist mit der Maßgabe, daß es sich bei der Teilsequenz nicht um die Sequenz MIKYNKIILTLTLLASLLAAC oder Teile hiervon handelt.

2. Immunologisch aktives Protein nach Anspruch 1, **dadurch gekennzeichnet, daß** die Teilsequenz des Proteins 1829-22B wenigstens 25 aufeinanderfolgende Aminosäuren aufweist.

3. Immunologisch aktives Protein nach Anspruch 1, **dadurch gekennzeichnet, daß** die Teilsequenz des Proteins 1829-22B wenigstens 50 aufeinanderfolgende Aminosäuren aufweist.

4. Testkit zum Nachweis von Antikörpern gegen Borrelia-Stämme, **dadurch gekennzeichnet, daß** es wenigstens ein immunologisch aktives Protein nach einem der Ansprüche 1 bis 3 enthält, das mit den in der Untersuchungsflüssigkeit vorhandenen Antikörpern reagieren kann und, daß es wenigstens eine Anzeigekomponente aufweist, die den Nachweis von Komplexen aus immunologisch aktivem Protein und Antikörper ermöglicht.

5. Testkit nach Anspruch 4, **dadurch gekennzeichnet, daß** es wenigstens ein immunologisch aktives Protein mit wenigstens einer Teilsequenz des Proteins 1829-22A und wenigstens ein Protein mit wenigstens einer Teilsequenz des Proteins 1829-22B aufweist.

6. Testkit nach einem der Ansprüche 4 oder 5, **dadurch gekennzeichnet, daß** die Anzeigekomponente ein gegen den nachzuweisenden Antikörper gerichteter Antikörper ist, der eine Markierung aufweist.

7. Testkit nach Anspruch 6, **dadurch gekennzeichnet, daß** die Anzeigekomponente ein Antihuman-IgG- oder Antihuman-IgM-Antikörper ist.

8. Testkit nach einem der Ansprüche 6 oder 7, **dadurch gekennzeichnet, daß** die Markierung aus einem Enzym besteht, das eine Farbreaktion katalysieren kann.

9. Testkit nach einem der Ansprüche 4 oder 5, **dadurch gekennzeichnet, daß** das immunologisch aktive Protein oder ein dagegen gerichteter monoklonaler Antikörper biotinyliert ist und, daß die Anzeigekomponente Avidin oder Streptavidin mit daran kovalent gebundenem Enzym, insbesondere Peroxidase ist.

10. Testkit nach einem der Ansprüche 4 bis 6, **dadurch gekennzeichnet, daß** es ein ELISA-Testkit ist.

11. Testkit nach Anspruch 10, **dadurch gekennzeichnet, daß** wenigstens ein immunologisch aktives Protein nach einem der Ansprüche 1 bis 3 an Mikrotiterplatten gekoppelt ist und die Anzeigekomponente aus Anti-Human-Immunoglobulin, insbesondere IgG- und/oder IgM-Antikörpern besteht, an die ein eine Farbreaktion katalysierendes Enzym gekoppelt ist.

12. Testkit nach Anspruch 4, **dadurch gekennzeichnet, daß** es ein Western-Blot ist.

13. Impfstoff, **dadurch gekennzeichnet, daß** er wenigstens ein Protein nach einem der Ansprüche 1 bis 3 enthält.

14. Verwendung eines immunologisch aktiven Proteins von Borrelia burgdorferi nach einem der Ansprüche 1 bis 3 zur Herstellung eines Impfstoffs zum Schutz gegen Infektionen von Borrelia burgdorferi-Bakterien.

15. Nukleinsäure, **dadurch gekennzeichnet, daß** sie für ein immunologisch aktives Protein nach einem der Ansprüche 1 bis 3 kodiert.

16. Nukleinsäure nach Anspruch 15, **dadurch gekennzeichnet, daß** sie eine DNA-Sequenz kodierend für das Protein 1829-22B mit der Sequenz oder eine Teilsequenz davon, die wenigstens 18 Nukleotide umfaßt, aufweist.

17. Nukleinsäure nach Anspruch 16, **dadurch gekennzeichnet, daß** die Teilsequenz wenigstens 30 Nukleotide umfaßt.

18. Verwendung einer Nukleinsäure nach einem der Ansprüche 15 bis 17 zum Nachweis einer Infektion von Borrelia burgdorferi mit Hilfe der Polymerase-Kettenreaktion.

19. Nukleinsäure nach einem der Ansprüche 15 bis 17 zur Verwendung in der DNA-Vaccinierung.

## Claims

1. An immunologically active protein from Borrelia burgdorferi which is present in a form which is free of other proteins derived from Borrelia burgdorferi, which protein exhibits the sequence of the protein 1829-22B, having the amino acid sequence or a partial sequence thereof having at least 10 consecutive amino acids, with the proviso that the partial sequence is not the sequence MIKYNKIILTLTLLASLLAAC or parts thereof.

2. An immunologically active protein as claimed in claim 1, wherein the partial sequence of protein 1829-22B exhibits at least 25 consecutive amino acids.

3. An immunologically active protein as claimed in claim 1, wherein the partial sequence of protein 1829-22B exhibits at least 50 consecutive amino acids.

4. A test kit for detecting antibodies against Borrelia strains, which contains at least one immunologically active protein as claimed in one of claims 1 to 3 which is able to react with the antibodies which are present in the fluid under investigation, and which contains at least one reporter component which makes it possible to detect complexes consisting of immunologically active protein and antibody.

5. A test kit as claimed in claim 4, which contains at least one immunologically active protein having at least a partial sequence of protein 1829-22A and at least one protein having at least a partial sequence of protein 1829-22B.

6. A test kit as claimed in claim 4 or 5, wherein the reporter component is an antibody which is directed against the antibody to be detected and which exhibits a label.

7. A test kit as claimed in claim 6, wherein the reporter component is an anti-human IgG antibody or an anti-human IgM antibody.

8. A test kit as claimed in claim 6 or 7, wherein the label consists of an enzyme which is able to catalyze a color reaction.

9. A test kit as claimed in claim 4 or 5, wherein the immunologically active protein, or a monoclonal antibody which is directed against it, is biotinylated, and wherein the reporter component is Avidin or Streptavidin to which an enzyme, in particular peroxidase, is covalently bonded.

10. A test kit as claimed in one of claims 4 to 6, which is an ELISA test kit.

11. A test kit as claimed in claim 10, wherein at least one immunologically active protein as claimed in one of claims 1 to 3 is coupled to microtiter plates, and the reporter component consists of anti-human immunoglobulin, in particular anti-IgG and/or anti-IgM antibodies to which an enzyme which catalyzes a color reaction is coupled.

12. A test kit as claimed in claim 4, which is a Western blot.

13. A vaccine, which contains at least one protein as claimed in one of claims 1 to 3.

14. The use of an immunologically active protein from Borrelia burgdorferi, as claimed in one of claims 1 to 3, for preparing a vaccine for protecting against infections with Borrelia burgdorferi bacteria.

15. A nucleic acid, which encodes an immunologically active protein as claimed in one of claims 1 to 3.

16. A nucleic acid as claimed in claim 15, which exhibits a DNA sequence encoding protein 1829-22B and having the sequence or a partial sequence thereof which encompasses at least 18 nucleotides.

17. A nucleic acid as claimed in claim 16, wherein the partial sequence encompasses at least 30 nucleotides.

18. The use of a nucleic acid as claimed in one of claims 15 to 17 for detecting an infection with Borrelia burgdorferi using the polymerase chain reaction.

19. A nucleic acid as claimed in one of claims 15 to 17 for use in DNA vaccination.

## Revendications

1. Protéine immunologiquement active de Borrelia burgdorferi, qui se présente sous une forme exempte d'autres protéines originaires Borrelia burgdorferi, **caractérisée en ce qu'**elle présente la séquence de la protéine 1829-22B avec la séquence d'acides aminés ou une séquence partielle de celle-ci présentant au moins 10 acides aminés successifs à la condition que, pour ce qui concerne la séquence partielle, il ne s'agisse pas de la séquence MIKYNKIILTLTLLASLLAAC ou de parties de celle-ci.

2. Protéine immunologiquement active suivant la revendication 1, **caractérisée en ce que** la séquence partielle de la protéine 1829-22B présente au moins 25 acides aminés successifs.

3. Protéine immunologiquement active suivant la revendication 1, **caractérisée en ce que** la séquence partielle de la protéine 1829-22B présente au moins 50 acides aminés successifs.

4. Trousse d'essai pour déceler des anticorps dirigés contre des souches de Borrelia, **caractérisée en ce qu'**elle contient au moins une protéine immunologiquement active suivant l'une des revendications 1 à 3, qui peut réagir avec les anticorps présents dans le liquide d'examen et **en ce qu'**elle présente au moins un composant indicateur qui permet la décèlement de complexes de protéine immunologiquement active et d'anticorps.

5. Trousse d'essai suivant la revendication 4, **caractérisée en ce qu'**elle présente au moins une protéine immunologiquement active comportant au moins une séquence partielle de la protéine 1829-22A et au moins une protéine comportant au moins une séquence partielle de la protéine 1829-22B.

6. Trousse d'essai suivant l'une des revendications 4 et 5, **caractérisée en ce que** le composant indicateur est un anticorps qui est dirigé contre l'anticorps à déceler et qui présente un marquage.

7. Trousse d'essai suivant la revendication 6, **caractérisée en ce que** le composant indicateur est un anticorps IgG antihumain ou un anticorps IgM antihumain.

8. Trousse d'essai suivant l'une des revendications 6 et 7, **caractérisée en ce que** le marquage est constitué d'une enzyme qui peut catalyser une réaction colorée.

9. Trousse d'essai suivant l'une des revendications 4 et 5, **caractérisée en ce que** la protéine immunologiquement active ou un anticorps monoclonal dirigé contre elle est biotinylé et **en ce que** le composant indicateur est de l'avidine ou de la streptavidine avec une enzyme fixée sur elle de manière covalente, par exemple de la peroxydase.

10. Trousse d'essai suivant l'une des revendications 4 à 6, **caractérisée en ce qu'**elle est une trousse d'essai ELISA.

11. Trousse d'essai suivant la revendication 10, **caractérisée en ce qu'**au moins une protéine immunologiquement active suivant l'une des revendications 1 à 3 est couplée à des plaques de microtitration et **en ce que** le composant indicateur est constitué d'immunoglobuline antihumaine, en particulier d'anticorps IgG et/ou d'anticorps IgM, sur laquelle est couplée une enzyme catalysant une réaction colorée.

12. Trousse d'essai suivant la revendication 4, **caractérisée en ce qu'**elle est un western-blot.

13. Vaccin, **caractérisé en ce qu'**il contient au moins une protéine suivant l'une des revendications 1 à 3.

14. Utilisation d'une protéine immunologiquement active de Borrelia burgdorferi suivant l'une des revendications 1 à 3, pour la fabrication d'un vaccin destiné à la protection vis-à-vis d'infections par des bactéries Borrelia burgdorferi.

15. Acide nucléique, **caractérisé en ce qu'**il code pour une protéine immunologiquement active suivant l'une des revendications 1 à 3.

16. Acide nucléique suivant la revendication 15, **caractérisé en ce qu'**il présente une séquence d'ADN codant pour la protéine 1829-22B, avec la séquence ou une séquence partielle de celle-ci qui comporte au moins 18 nucléotides.

17. Acide nucléique suivant la revendication 16, **caractérisé en ce que** la séquence partielle comporte au moins 30 nucléotides.

18. Utilisation d'un acide nucléique suivant l'une des revendications 15 à 17, pour déceler une infection par Borrelia burgdorferi à l'aide d'une amplification en chaîne par polymérase.

19. Acide nucléique suivant l'une des revendications 15 à 17, à utiliser dans la vaccination ADN.
